# EUROPÄISCHE PATENTSCHRIFT

(11) **EP 1 138 340 B1**
(45) Veröffentlichungstag und Bekanntmachung des Hinweises auf die Patenterteilung: **15.12.2004**
(21) Anmeldenummer: 01105435.0
(22) Anmeldetag: 13.03.2001
(51) Int. Cl.: A61M 16/08

(54) **Vorrichtung zur Ableitung von Atemgas.**
Device for evacuating respiratory gas
Dispositif pour l'évacuation de gaz respiratoire

(30) Priorität: 23.03.2000 DE 10014178
(43) Veröffentlichungstag der Anmeldung: 04.10.2001
(73) Patentinhaber: MAP Medizin-Technologie GmbH, 82152 Martinsried (DE)
(72) Erfinder: Brandmeier, Richard, 86931 Prittriching (DE); Madaus, Stefan, 82152 Krailling (DE); Vögele, Harald, 82131 Gauting (DE); Lang, Bernd, 82166 Gräfelfing (DE)
(74) Vertreter: Rössig, Rolf

(56) Entgegenhaltungen:
- EP-A- 0 697 225
- EP-A- 0 985 430
- WO-A-98/34665
- DE-C- 19 757 703
- US-A- 5 762 382
- US-A- 5 937 851

## Beschreibung

Die Erfindung betrifft eine Vorrichtung zur Ableitung von zumindest teilweise verbrauchtem Atemgas aus einem Atemgasleitungssystem. Derartige Vorrichtungen finden insbesondere Anwendung im Bereich der Schlafmedizin. So kann beispielsweise schlafbezogenen Atmungsstörungen durch eine auch als CPAP-Therapie bezeichnete Überdruckbeatmung vorgebeugt werden.

Bei bekannten CPAP-Gerätesystemen ist üblicherweise eine Atemgas-Fördereinrichtung vorgesehen, die über eine flexible Schlauchleitung mit einer patientenseitig angeordneten Atemmaske gekoppelt ist.

Im Rahmen eines Inspirationsvorganges nimmt der Patient das in der flexiblen Schlauchleitung unter Überdruck bereit gehaltene und ständig nachgeförderte Atemgas auf. Während eines Expirationsvorganges wird das ausgeatmete Atemgas zumindest teilweise in die flexible Schlauchleitung zurückgeführt. Um eine ständige Nachfuhr frischen Atemgases zu gewährleisten, ist vorzugsweise im Bereich der Atemmaske eine Vorrichtung zur Ableitung eines definierten Atemgasstromes vorgesehen, über welche ein permanenter Abstrom des unter einem vorbestimmten Überdruck stehenden Atemgases in die Umgebung erfolgen kann. Eine derartige Vorrichtung ist aus der auf die Anmelderin zurückgehenden, veröffentlichten deutschen Patentanmeldung DE 198 40 760.2 bekannt. Diese Vorrichtung besteht aus zwei Rohrelementen, die miteinander unter Verwendung eines Schnappringes lösbar in Steckverbindungskontakt bringbar sind. Über einen zwischen diesen beiden Rohrelementen gebildeten schmalen Ringspalt kann ein definierter Gasstrom aus dem Leitungsbereich abgezweigt werden.

Das Dokument US-A-5 937 851 offenbart eine Vorrichtung zur Ableitung von verbrauchtem Atemgas gemäß dem Oberbegriff des Anspruchs 1.

Der Erfindung liegt die Aufgabe zugrunde, eine Vorrichtung zur Ableitung von zumindest teilweise verbrauchtem Atemgas zu schaffen, die sich bei verbleichbar einfacher Handhabung durch eine nochmals geringere Geräuschemission auszeichnet.

Diese Aufgabe wird erfindungsgemäß gelöst durch eine Vorrichtung gemäß Anspruch 1.

Dadurch wird es auf vorteilhafte Weise möglich, eine auch unter fertigungstechnischen Gesichtspunkten günstig herstellbare Vorrichtung zu schaffen, welche sich durch eine vergleichsweise geringe Schallemission bei der Ableitung des verbrauchten Atemgases auszeichnet.

Gem. einer besonders bevorzugten Ausführungsform der Erfindung ist der erste Anschlußabschnitt aus einem Silikonkautschukmaterial gebildet. Hierdurch wird es möglich, den ersten Anschlußabschnitt unter elastischer Verformung fest sitzend mit einem weiteren Organ des Atemgasleitungssystems zu koppeln. Insbesondere unmittelbar auf einen Anschlußstutzen einer Atemmaske aufzustecken.

In vorteilhafter Weise ist auch der zweite Anschlußabschnitt aus einem Silikonkautschukmaterial gebildet. Hierdurch wird es möglich, auch die weitere Kopplung der Vorrichtung mit dem entsprechenden Abschnitt des Atemgasleitungssystems auf zuverlässige Weise und mit hoher Dichtwirkung vorzunehmen. Insbesondere für den Fall, daß beide Anschlußabschnitte aus dem Silikonmaterial gebildet sind, ergibt sich ein besonders geringer Eintrag von Körperschall in das Atemgasleitungssystem.

Eine unter fertigungstechnischen Gesichtspunkten besonders günstig realisierbare Ausführungsform der Erfindung ist dadurch gegeben, daß die Kanaleinrichtung im Bereich einer Fügezone zwischen den beiden Anschlußabschnitten ausgebildet ist. Die Kanaleinrichtung kann hierbei durch eine Vielzahl von rinnenartigen Vertiefungen gebildet sein, die bei entsprechender Koppelung der beiden Anschlußabschnitte rohrleitungsartige Kanäle bilden. Gem. einer besonders bevorzugten Ausführungsform der Erfindung ist der Verlauf der Kanäle derart gewählt, daß sich ein vergleichsweise hoher Strömungswiderstand ergibt. In vorteilhafter Weise ist im Austrittsbereich der jeweiligen Kanaleinrichtung eine Diffusorzone vorgesehen, innerhalb welcher die Strömungsgeschwindigkeit des abströmenden Gases abgesenkt wird.

Gem. einer besonders bevorzugten Ausführungsform der Erfindung sind die beiden Anschlußabschnitte zueinander drehbar gelagert. Alternativ dazu ist es auch möglich, eine Drehmuffeneinrichtung vorzusehen, über welche eine Relativdrehung der unmittelbar an die Vorrichtung angeschlossenen Leitungsorgane zueinander zugelassen ist.

Gem. einer besonders bevorzugten Ausführungsform der Erfindung ist weiterhin eine elastomere Kippstruktur vorgesehen, die eine Verkippung der an die Vorrichtung angeschlossenen Leitungsorgane zueinander zuläßt. Diese Kippstruktur kann beispielsweise durch eine Ringbalgstruktur erreicht werden, die in wenigstens einem der Anschlußabschnitte ausgebildet ist.

Die Ableitungseinrichtung umfaßt vorzugsweise eine Vielzahl von in den elastomeren Körper eingeformten Kanälen. Die Kanäle können einen labyrinthartigen Verlauf aufweisen, wodurch ein noch wirkungsvollerer Druckabbau des abströmenden Atemgases erreicht wird. Die Kanäle können in gleichmäßiger Teilung in Umfangsrichtung angeordnet sein. Alternativ dazu ist es auch möglich, in bestimmten Umfangszonen keine Kanäle auszubilden, so daß beispielsweise eine Abstrahlung des abgeleiteten Atemgases auf die Gesichtsfläche des Patienten vermieden ist. Die Abstrahlung des Atemgases erfolgt im wesentlichen parallel zu einer gemeinsamen Längsachse der beiden Anschlußabschnitte. Altemativ dazu ist es auch möglich, die Abstrahlung entlang eines Kegelmantels oder zumindest in einem innerhalb eines Kegelmantels liegenden Bereich vorzunehmen.

Die Ableitungseinrichtung ist vorzugsweise derart dimensioniert, daß hieraus ein Atemgasstrom im Bereich von 20 bis 60 l / min abströmen kann. Dies kann beispielsweise erreicht werden, indem der gesamte Minimalquerschnitt der Ableitungseinrichtung im Bereich von 8 bis 20 mm² liegt. Bei der Verwendung von Labyrinthstrukturen ist es möglich, größere Querschnitte zuzulassen. Durch größere Querschnitte wird in vorteilhafter Weise einer ungewünschten Verstopfung dieser Kanäle vorgebeugt. Auch wird die Reinigung dieser Kanäle erleichtert.

Die Kanäle können einen runden oder polygonalen Querschnitt aufweisen. Vorzugsweise sind die Kanäle derart ausgebildet, daß diese sich im wesentlichen entsprechend der Zunahme der Strömungsgeschwindigkeit verjüngen. Zur Reduzierung der Abströmungsgeschwindigkeit ist es möglich, die Abstrahlung des Atemgases über eine Diffusorzone oder in eine Staudruckzone hinein vorzunehmen.

Bevorzugte Ausgestaltungen der Erfindung sind Gegenstand der Unteransprüche.

Weitere Einzelheiten und Merkmale der Erfindung ergeben sich aus der nachfolgenden Beschreibung mehrerer bevorzugter Ausführungsbeispiele in Verbindung mit der Zeichnung. Es zeigen:
- **Fig. 1**: eine vereinfachte Axialschnittansicht einer Ausführungsform einer Ableitungseinrichtung, bei welcher sowohl der erste Anschlußabschnitt als auch der zweite Anschlußabschnitt aus einem elastomeren Material, hier Silikonkautschuk, gebildet ist und die Kanaleinrichtung sich im Bereich einer zwischen den beiden Anschlußabschnitten verlaufenden Fügestelle befindet;
- **Fig. 2**: ebenfalls eine Axialschnittansicht, wobei jedoch nur einer der Anschlußabschnitte aus einem elastomeren Material gebildet ist und der zweite Anschlußabschnitt lediglich durch eine drehbar aufgesteckte Muffe verwirklicht ist;
- **Fig. 3**: eine Axialschnittansicht einer weiteren Ausführungsform einer Ableitungseinrichtung mit einem aus einem Elastomermaterial gefertigten Anschlußabschnitt und einem darin eingesteckten zweiten Anschlußabschnitt mit einer drehbar gelagerten Schlauchanschlußmuffe;
- **Fig. 4**: eine weitere Ausführungsform einer Ableitungsvorrichtung mit einem glokkenförmigen Gehäuseteil, in welches ein aus einem Elastomermaterial gebildeter Anschlußabschnitt eingesteckt ist;

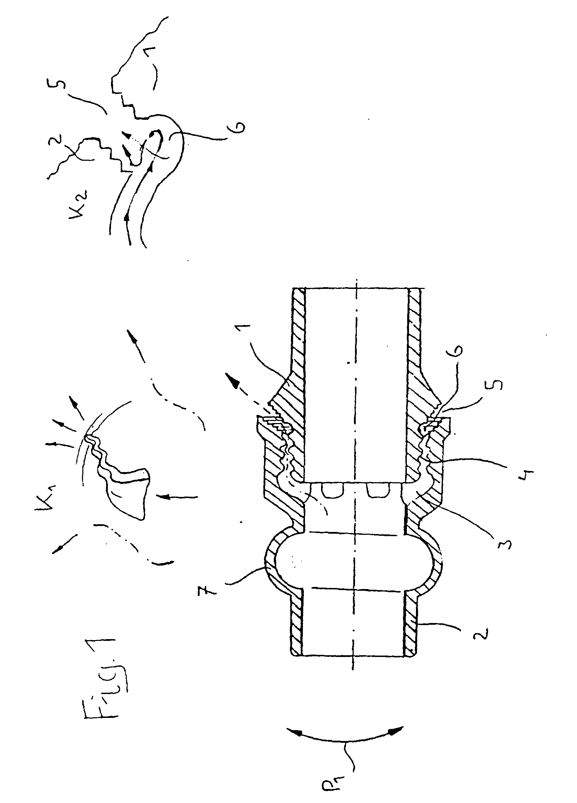

Die in Fig. 1 gezeigte Vorrichtung umfaßt einen ersten Anschlußabschnitt 1 und einen zweiten Anschlußabschnitt 2, die hier beide aus einem Elastomermaterial gebildet sind. Die beiden Anschlußabschnitte 1, 2 stehen miteinander über eine Umfangsnutenstruktur 4 in Eingriff. Diese Umfangsnutenstruktur 4 umfaßt eine seitens des ersten Anschlußabschnitts ausgebildete Umfangsprofilierung sowie eine hierzu komplementär ausgebildete, am zweiten Anschlußabschnitt 2 vorgesehene Innenprofilierung. Die Eingriffsstruktur gelangt im Wege elastischer Verformung in Raststellung. Im Bereich der Fügestelle zwischen den beiden Anschlußabschnitten 1, 2 ist eine Ableitungseinrichtung 3 vorgesehen, die hier mehrere in Umfangsrichtung voneinander beabstandete Kanäle aufweist. Die Kanäle sind hier durch rinnenartige Ausnehmungen im Bereich der Innenfläche des zweiten Anschlußabschnittes 2 gebildet. Der durch die Kanäle definierte Innenraum ist beispielshaft in der oberen Skizze K₁ angedeutet. Der hier erkennbare wellenartige Verlauf jedes Kanals ist insbesondere durch die Profilierung der Außenumfangsfläche des ersten Anschlußabschnitts 1 im Bereich der Fügestelle bestimmt.

Im Austrittsbereich 5 der Kanäle ist eine sich erweiternde, in einer Umfangsringnut gebildete Diffusorzone vorgesehen, die mit einer Vielzahl feiner Stufen versehen ist, durch welche der Strömungswiderstand der Diffusorzone noch weiter gesteigert wird. Die Befüllung der Diffusorzone im Bereich der äußeren Mündungen der Kanäle erfolgt derart gerichtet, daß im innenliegenden Fußbereich 6 des Diffusors eine Staudruckzone entsteht. Eine hierzu geeignete Querschnittsgestaltung, die auch bei der dargestellten Ausführungsform Anwendung findet, ist in der Skizze K₂ gezeigt. Die obere Wandung ist hier durch den zweiten Anschlußabschnitt 2 und die untere Wandung durch den ersten Wandungsabschnitt 1 gebildet. Einer der beiden Anschlußabschnitte, hier der zweite, ist mit einer Balgstruktur 7 versehen, durch welche ein Verkippen der beiden Anschlußabschnitte 1, 2, wie durch den Pfeil P₁ angedeutet, möglich ist.

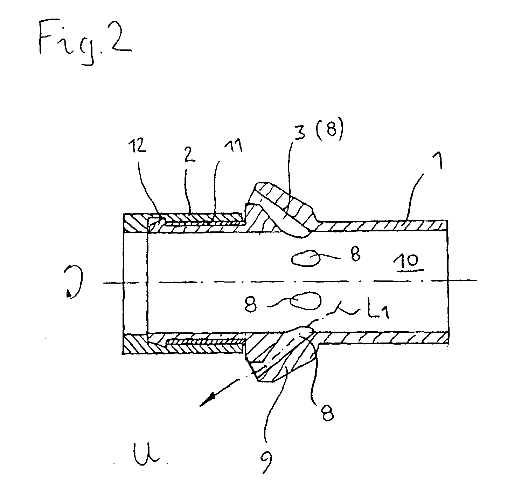

Bei der in Fig. 2 dargestellten Ausführungsform ist die Ableitungseinrichtung 3 durch mehrere Kanäle 8 gebildet, die sich im Bereich eines radial nach außen vorragenden Wulstes 9 nach außen erstrecken und eine Leitungsverbindung zwischen dem Innenbereich 10 und der Umgebung U schaffen. Die Abströmung des Atemgases erfolgt hierbei wie durch die Strichpunktlinie L₁ angedeutet. Die Kanäle 8 sind hier derart ausgebildet, daß sich diese entsprechend der Zunahme der Strömungsgeschwindigkeit zunächst verjüngen und dann anschließend in eine Diffusorzone einmünden. Der hier radial nach außen vorragende Umfangswulst ist integral mit dem ersten Anschlußabschnitt 1 aus einem Elastomermaterial gebildet. Um eine Relativdrehbarkeit der durch die Vorrichtung miteinander gekoppelten Leitungsorgane zu ermöglichen, ist der zweite Anschlußabschnitt 2 durch eine Drehmuffenanordnung gebildet, die drehbar auf den ersten Anschlußabschnitt 1 aufgesetzt ist. Um eine besonders leichtgängige Drehbewegung zu gewährleisten, ist hierzu ein Ringelement 11 vorgesehen, das vorzugsweise aus einem hochfesten Material - insbesondere Edelstahl gebildet ist. Die Koppelung des zweiten Anschlußabschnitts 2 mit dem ersten Anschlußabschnitt 1 erfolgt hier über einen Rastmechanismus 12, der derart abgestimmt ist, daß der zweite Anschlußabschnitt 2 unter Aufbringung einer definierten Abzugskraft von dem ersten Anschlußabschnitt 1 abgezogen werden kann.

Die Ausrichtung der Kanäle 8 ist hier derart getroffen, daß die Abstrahlung des abgeleiteten Atemgases entlang eines Kegelmantels erfolgt. Der zweite Anschlußabschnitt 2 ist hier aus einem Kunststoffmaterial, beispielsweise Polycarbonat, gebildet.

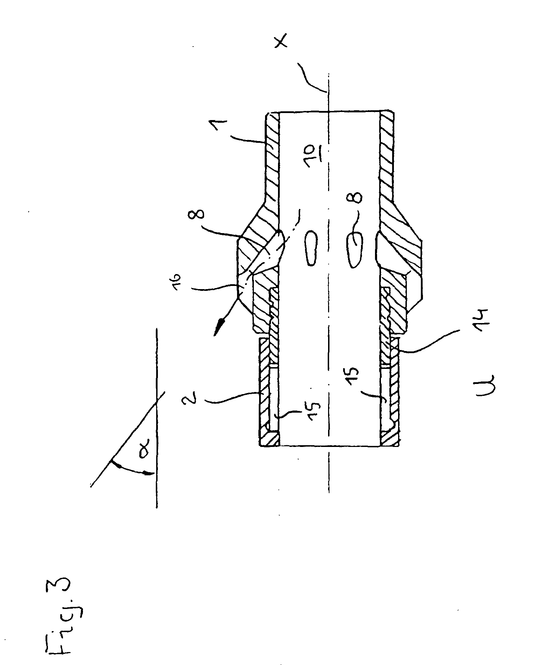

In Fig. 3 ist eine weitere Ausführungsform einer erfindungsgemäßen Vorrichtung dargestellt, bei welcher, ähnlich, wie bei der Ausführungsform gem. Fig. 2 eine Abstrahlung der abgeleiteten Luft entlang eines Kegelmantels erfolgt. Die Kanäle 8 verbinden hier ebenfalls den Innenbereich 10 mit der Umgebung U. Ein wesentlicher Unterschied gegenüber der Ausführungsform gem. Fig. 2 besteht hier in der Ausgestaltung der Drehmuffeneinrichtung, durch welche eine Relativdrehbarkeit des zweiten Anschlußabschnitts 2 gegenüber dem ersten Anschlußabschnitt 1 erreicht wird. Bei der hier dargestellten Ausführungsform ist hierzu ein Lagerzapfenelement 14 vorgesehen, das in abdichtender Weise in einen entsprechenden Aufnahmeabschnitt des ersten Anschlußabschnittes 1 eingesteckt ist. An dem Lagerzapfenelement 14 sind mehrere radial nachgiebige Rastzungenelemente 15 vorgesehen, die lösbar mit dem hier rohrbuchsenartig ausgebildeten zweiten Anschlußabschnitt 2 in Eingriff bringbar sind.

Bei der hier dargestellten Ausführungsform wird die Intensität der auftretenden Geräuschereignisse durch eine Lamellenstruktur 16 noch weiter reduziert, in welche die Kanäle 8 einmünden. Die Kanäle 8 weisen hier einen im wesentlichen kreisförmigen Querschnitt auf und sind schräg zu einer Längsmittelachse X der Vorrichtung ausgerichtet. Der gegenüber der Längsmittelachse X definierte Abstrahlwinkel α bewegt sich vorzugsweise im Bereich von 10 bis 50°. Die gesamte Querschnittsfläche im Bereich der engsten Stellen der Kanäle 8 beträgt bei der hier dargestellten Ausführungsform 21 mm².

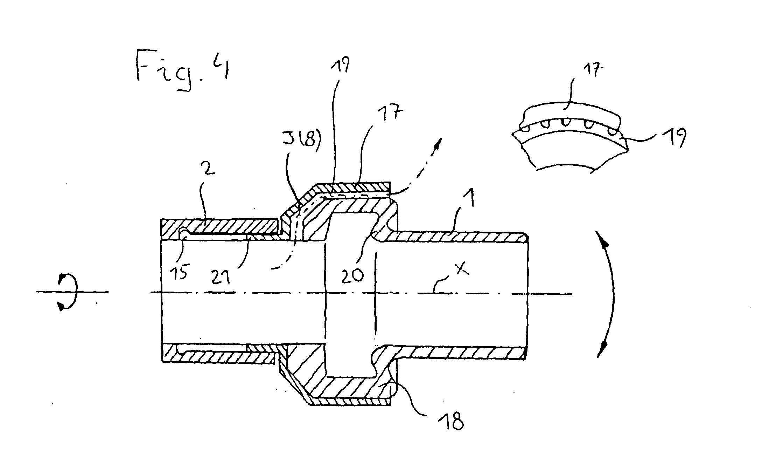

In Fig. 4 ist eine weitere Ausführungsform der erfindungsgemäßen Vorrichtung gezeigt, bei welcher die Kanaleinrichtung 3 im Zusammenspiel eines glockenförmigen Gehäuseteils 17 mit einem darin eingesteckten Elastomerkörper 18 gebildet ist. Der Elastomerkörper 18 ist hier integral mit dem ersten Anschlußabschnitt 1 ausgebildet und weist mehrere in Umfangsrichtung voneinander beabstandete Rillen 19 auf. Die Rillen 19 werden nach außen hin von dem glockenförmigen Gehäuseteil 17 begrenzt und bilden hierbei entsprechende Kanäle 8. Die Koppelung des elastomeren Körpers 18 mit dem Anschlußabschnitt 1 erfolgt über eine Balgstruktur 20, die ein Verkippen des ersten Anschlußabschnitts 1 gegenüber dem zweiten Anschlußabschnitt 2 ermöglicht. Der zweite Anschlußabschnitt 2 ist hier um die Längsachse X relativ zum ersten Anschlußabschnitt drehbar. Die Drehlagerung des zweiten Anschlußabschnitts 2 erfolgt über einen Buchsenabschnitt 21, welcher Rastarme 15 aufweist, die lösbar mit einer entsprechend komplementären Innenumfangsstruktur des zweiten Anschlußabschnitts 2 in Eingriff bringbar sind. Die Gesamtquerschnittsfläche der Kanäle 8 (hier insgesamt 19 Stück) beträgt 16 mm².

Anhand eines praktischen Anwendungsbeispieles wird nachfolgend die Funktionsweise der erfindungsgemäßen Vorrichtung noch näher beschrieben. Zur Durchführung einer CPAP-Therapie zur Behandlung schlafbezogener Atmungsstörungen wird ein CPAP-Gerät neben dem Bett eines Patienten plaziert. An das CPAP-Gerät wird eine flexible Schlauchleitung angeschlossen. An das dem CPAP-Gerät abgewandte Ende der Schlauchleitung wird eine Vorrichtung zur Ableitung von zumindest teilweise verbrauchtem Atemgas angeschlossen, wie sie insbesondere in Fig. 3 dargestellt ist. Hierzu wird der zweite Anschlußabschnitt 2 in das entsprechende Ende der flexiblen Schlauchleitung eingesteckt. Anschließend wird der erste Anschlußabschnitt 1 auf einen entsprechenden Anschlußabschnitt einer Atemmaske aufgesteckt. Die Atemmaske kann nunmehr beispielsweise über eine Kopfbandanordnung auf dem Nasenbereich des Patienten plaziert und fixiert werden. Aufgrund der zwischen dem zweiten Anschlußabschnitt 2 und dem ersten Anschlußabschnitt 1 vorgesehenen Drehmechanismuns wird eine unmittelbare Übertragung etwaiger Drehmomente aus der flexiblen Schlauchleitung in die Atemmaske vermieden. Durch das CPAP-Gerät wird permanent Atemgas, beispielsweise gefilterte Umgebungsluft, unter einem vorbestimmten Druck in die flexible Schlauchleitung hinein gefördert. Das Atemgas durchströmt die flexible Schlauchleitung und gelangt hierbei in die Atemmaske. Das Atemgas kann nunmehr von dem Patienten eingeatmet und anschliessend ausgeatmet werden. Sowohl während des Einatmungsvorganges als auch während des Ausatmungsvorganges strömt permanent Luft über die in der zwischen Schlauchleitung und Maske vorgesehene Vorrichtung, insbesondere über die hierin ausgebildeten Kanäle 8 ab. Durch die permanente Abströmung des Atemgases aus den Kanälen wird eine permanente Spülung des Atemgases im Bereich der Atemmaske erreicht. Die Abstrahlung des abgeleiteten Atemgases über die Kanäle 8 erfolgt derart gerichtet, daß der abströmende Luftstrom nicht auf den Stirnbereich des Patienten auftrifft. Die Abstrahlung des abgeleiteten Atemgases erfolgt besonders geräuscharm, da neben einer besonders günstigen Leitungsführung auch der Eintrag von Körperschall in das Atemgasleitungssystem erheblich reduziert ist.

## Patentansprüche

1. Vorrichtung zur Ableitung von zumindest teilweise verbrauchtem Atemgas aus einem Atemgasleitungssystem mit:
einer zur Anordnung zwischen einer Atemmaske und einer Schlauchleitung vorgesehenen Leitungseinrichtung,
wobei die Leitungseinrichtung
einen ersten Anschtussabschnitt (1) und
einen zweiten Anschlussabschnitt (2) aufweist, und mit
einer Ableitüngseinrichtung (3) versehen ist, zur Ableitung eines Atemgasteilstromes,
**dadurch gekennzeichnet dass** wenigstens einer der Anschiussabschnitte (1, 2) als aus einem elastomeren Material gefertigter Körper ausgebildet ist, und die Ableitungseinrichtung wenigstens einen Kanal (8) aufweist, dessen Wandung zumindest teilweise durch den elastomeren Körper begrenzt ist.

2. Vorrichtung nach Anspruch 1, **dadurch gekennzeichnet, daß** eine Koppelungseinrichtung (12,15) vorgesehen ist, zur Koppelung der beiden Anschlußabschnitte (1, 2) in lösbarer Weise.

3. Vorrichtung nach Anspruch 1 oder 2, **dadurch gekennzeichnet, daß** der erste Anschlußabschnitt (1) aus einem Silikonkautschukmaterial gebildet ist.

4. Vorrichtung nach einem der Ansprüche 1 bis 3, **dadurch gekennzeichnet, daß** der zweite Anschlußabschnitt (2) aus einem Silikonkautschukmaterial gebildet ist.

5. Vorrichtung nach Anspruch 1 oder 2, **dadurch gekennzeichnet, daß** beide Anschlußabschnitte (1, 2) aus einem Silikonkautschukmaterial gebildet sind.

6. Vorrichtung nach wenigstens einem der Ansprüche 1 bis 5, **dadurch gekennzeichnet, daß** die Kanaleinrichtung (8) im Bereich einer Fügezone verläuft.

7. Vorrichtung nach wenigstens einem der Ansprüche 1 bis 6, **dadurch gekennzeichnet, daß** eine Drehmuffeneinrichtung (11,21) vorgesehen ist, zur drehbaren Koppelung der Anschlußabschnitte (1, 2);

8. Vorrichtung nach einem der Ansprüche 1 bis 7, **dadurch gekennzeichnet, daß** eine elastomere Kippstruktur (20) vorgesehen ist, zum Ermöglichen einer Kippbewegung der beiden Anschlußabschnitte (1,2) gegeneinander.

9. Vorrichtung nach wenigstens einem der Ansprüche 1 bis 8, **dadurch gekennzeichnet, daß** die Ableitungseinrichtung (3) durch mehrere in den elastomeren Körper eingeformte Kanäle (8) gebildet ist.

10. Vorrichtung nach wenigstens einem der Ansprüche 1 bis 9, **dadurch gekennzeichnet, daß** die Ableitungseinrichtung (3) im Bereich einer Fügestelle gebildet ist.

11. Vorrichtung nach wenigstens einem der Ansprüche 1 bis 10, **dadurch gekennzeichnet, daß** die Ableitungseinrichtung (3) eine Labyrinthstruktur aufweist.

## Claims

1. Apparatus for discharging at least partially consumed respiratory gas from a respiratory gas conduit system comprising:
a conduit device provided for arrangement between a breathing mask and a hose conduit,
wherein the conduit device includes
a first connecting portion (1) and
a second connecting portion (2), and
is provided with a discharge device (3) for the discharge of a respiratory gas partial flow,
**characterised in that** at least one of the connecting portions (1, 2) is in the form of a body produced from an elastomer material, and the discharge device has at least one duct (8), the wall of which is at least partially delimited by the elastomer body.

2. Apparatus according to claim 1 **characterised in that** there is provided a coupling device (12, 15) for releasably coupling the two connecting portions (1, 2).

3. Apparatus according to claim 1 or claim 2 **characterised in that** the first connecting portion (1) is formed from a silicone rubber material.

4. Apparatus according to one of claims 1 to 3 **characterised in that** the second connecting portion (2) is formed from a silicone rubber material.

5. Apparatus according to claim 1 or claim 2 **characterised in that** both connecting portions (1, 2) are formed from a silicone rubber material.

6. Apparatus according to at least one of claims 1 to 5 **characterised in that** the duct arrangement (8) extends in the region of a joining zone.

7. Apparatus according to at least one of claims 1 to 6 **characterised in that** there is provided a rotary sleeve device (11, 21) for rotatably coupling the connecting portions (1, 2).

8. Apparatus according to one of claims 1 to 7 **characterised in that** there is provided an elastomer tilting structure (20) for permitting a tilting movement of the two connecting portions (1, 2) relative to each other.

9. Apparatus according to at least one of claims 1 to 8 **characterised in that** the discharge device (3) is formed by a plurality of ducts (8) formed in the elastomer body.

10. Apparatus according to at least one of claims 1 to 9 **characterised in that** the discharge device (3) is formed in the region of a join location.

11. Apparatus according to at least one of claims 1 to 10 **characterised in that** the discharge device (3) has a labyrinth structure.

## Revendications

1. Dispositif pour l'évacuation de gaz respiratoire au moins partiellement consumé à partir d'un système de conduite de gaz respiratoire comportant :
un dispositif de conduite prévu entre un masque respiratoire et une conduite en
tuyau,
le dispositif de conduite présentant
une première section de raccordement (1) et
une seconde section de raccordement (2), et étant pourvu d'un dispositif d'évacuation (3) pour l'évacuation d'un flux partiel de gaz respiratoire,
**caractérisé en ce qu'**au moins une des sections de raccordement (1, 2) est formée à partir d'un corps fabriqué en un matériau élastomère et que le dispositif d'évacuation comporte au moins un canal (8) dont la paroi est du moins partiellement limitée par le corps élastomère.

2. Dispositif selon la revendication 1, **caractérisé en ce qu'**un dispositif de raccordement (12, 15) est prévu pour raccorder les deux sections de raccordement (1, 2) de manière à ce qu'elles puissent être détachées.

3. Dispositif selon les revendications 1 ou 2, **caractérisé en ce que** la première section de raccordement (1) est formée dans un matériau de caoutchouc de silicone.

4. Dispositif selon l'une quelconque des revendications 1 à 3, **caractérisé en ce que** la seconde section de raccordement (2) est formée dans un matériau de caoutchouc de silicone.

5. Dispositif selon les revendications 1 ou 2, **caractérisé en ce que** les deux sections de raccordement (1, 2) sont formées dans un matériau de caoutchouc de silicone.

6. Dispositif selon au moins une des revendications 1 à 5, **caractérisé en ce que** le dispositif de canaux (8) s'étend au niveau d'une zone de joint.

7. Dispositif selon au moins une des revendications 1 à 6, **caractérisé en ce qu'**un dispositif de manchon rotatif (11, 21) est prévu pour le raccordement rotatif des sections de raccordement (1,2).

8. Dispositif selon l'une quelconque des revendications 1 à 6, **caractérisé en ce qu'**une structure élastomère à bascule (20) est prévue pour permettre un mouvement de bascule réciproque des deux sections de raccordement (1, 2).

9. Dispositif selon au moins une des revendications 1 à 8, **caractérisé en ce que** le dispositif d'évacuation (3) est formé par plusieurs canaux (8) pratiqués dans le corps élastomère.

10. Dispositif selon au moins une des revendications 1 à 9, **caractérisé en ce que** le dispositif d'évacuation (3) est formé au niveau d'un joint.

11. Dispositif selon au moins une des revendications 1 à 10, **caractérisé en ce que** le dispositif d'évacuation (3) présente une structure en labyrinthe.
